# FASCICULE DE BREVET EUROPEEN

(11) **EP 1 935 456 B1**
(45) Date de publication et mention de la délivrance du brevet: **10.11.2010**
(21) Numéro de dépôt: 07123807.5
(22) Date de dépôt: 20.12.2007
(51) Int. Cl.: A61Q 5/10, A61K 8/41, A61K 8/44, A61K 8/73

(54) **Composition de teinture d'oxydation comprenant un tensio-actif cationique, un biohétéropolysaccharide, un tensio-actif amphotère et un précurseur de colorant**
Zusammensetzung für das direkte Färben, die ein kationisches Tensid, ein Bioheteropolysaccharid, ein amphoteres Tensid und ein Farbstoffvorprodukt enthält
Oxidation dye composition containing a cationic surface-active agent, a bioheteropolysaccharide, an amphoteric surface-active agent and a dye precursor

(30) Priorité: 21.12.2006 FR 0655806
(43) Date de publication de la demande: 25.06.2008
(73) Titulaire: L'Oréal, 75008 Paris (FR)
(72) Inventeur: Huet, Nathalie, 75012, PARIS (FR); Audousset, Marie-Pascale, 92600, ASNIERES (FR)
(74) Mandataire: Fevrier, Murielle Françoise E.

(56) Documents cités:
- EP-A- 1 142 561
- EP-A- 1 279 395
- EP-A- 1 716 841
- EP-A1- 1 234 569
- WO-A-01/78668
- FR-A1- 2 575 067

## Description

La présente invention concerne une composition de teinture d'oxydation des fibres kératiniques, en particulier des fibres kératiniques humaines.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'associer ces précurseurs de colorants à des biohétéropolysaccharides. Notamment, la demande de brevet FR 2 575 067 décrit des compositions de teinture des fibres kératiniques comprenant un colorant d'oxydation et un bio-hétéropolysaccharide pour améliorer la solidité des nuances avec une bonne couvrance des cheveux blancs et une diminution de la sélectivité, cette composition pouvant contenir ou non des agents tensio-actifs.

Ces compositions ne donnent cependant pas des propriétés tinctoriales totalement satisfaisantes, notamment en ce qui concerne la sélectivité. Par ailleurs, la texture des produits ne donne pas totalement satisfaction ce qui nuit aux qualités d'usage du produit telles que la facilité de mélange avec les agents oxydants et/ou la facilité d'application des mélanges sur les fibres kératiniques.

Ainsi, le but de la présente invention est de développer de nouvelles compositions de teinture d'oxydation des fibres kératiniques telles que les cheveux qui permettent de résoudre ces problèmes techniques. En particulier, l'invention cherche à obtenir des compositions qui permettent d'obtenir des produits qui s'appliquent bien sur les fibres kératiniques et qui présentent des propriétés tinctoriales améliorées.

Ainsi, ce but est atteint avec la présente invention qui a pour objet une composition de coloration des fibres kératiniques comprenant au moins un précurseur de colorant, au moins un bio-hétéropolysaccharide, au moins un tensio-actif cationique et au moins un tensio-actif amphotère.

Une telle composition permet en particulier d'obtenir une facilité d'application ainsi que des propriétés tinctoriales améliorées, en particulier la sélectivité ce qui montre une amélioration de l'uniformité de la coloration sur la longueur des mèches.

La composition de l'invention peut contenir à titre de précurseur de colorant une base d'oxydation et/ou un coupleur. Selon un mode de réalisation particulier, la composition comprend au moins une base d'oxydation.

A titre d'exemple, la ou les bases d'oxydation sont choisies parmi les paraphénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la paraphénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro paraphénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy paraphénylènediamine, la N-(β-méthoxyéthyl) para-phénylène-diamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la paraphénylènediamine, la para-toluènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl paraphénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxypyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation utiles peuvent être des bases d'oxydation cationiques. On entend par base d'oxydation cationique celle qui porte au moins une charge cationique permanente.

Les bases d'oxydation cationiques utilisables selon l'invention peuvent être des para-phénylènediamines telles que notamment décrites dans les demandes de brevets FR 2 766 177 et FR 2 766 178, des para-aminophénols tels que décrits par exemple dans les demandes de brevets FR 2 766 177 et FR 2 766 178, des ortho-phénylènediamines telles que décrites par exemple dans les demandes de brevets FR 2 782 718, FR 2 782 716 et FR 2 782 719, des orthoaminophénols ou des bases hétérocycliques ou des bases doubles cationiques plus particulièrement telles que décrites dans les demandes de brevet FR 2 766 179. A titre d'exemple, on peut notamment citer des bases d'oxydation cationiques de structure paraphénylènediamine, dont une des fonctions amine est une amine tertiaire, porteur d'un noyau pyrrolidinique, la molécule possédant au moins un atome d'azote quaternisé. Ce type de bases est décrit dans le document FR 2 837 821.

A titre de bases hétérocycliques cationiques, on peut citer les bases pyrazoles cationiques ou des pyrazolopyrimidines cationiques par exemple décrites dans les documents FR 2 788 521 et FR 2 788 522.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

Parmi le ou les coupleurs, on peut notamment citer les méta-phénylènediamines, les méta-aminophénols, les méta-diphénols, les coupleurs naphtaléniques, les coupleurs hétérocycliques et leur sels d'addition.

Le ou les méta-aminophénols pouvant être utilisés à titre coupleur dans la composition tinctoriale prête à l'emploi conforme à l'invention sont de préférence choisis parmi les composés de formule (I) suivante, et leurs sels d'addition avec un acide : 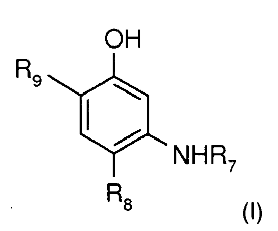 dans laquelle :
- R₇ représente un atome d'hydrogène, un radical alkyle en C₁-C₄, monohydroxyalkyle en C₁-C₄ ou polyhydroxyalkyle en C₂-C₄,
- R₈ représente un atome d'hydrogène, un radical alkyle en C₁-C₄, alcoxy en C₁-C₄ ou un atome d'halogène choisi parmi le chlore, le brome ou le fluor,
- R₉ représente un atome d'hydrogène, un radical alkyle en C₁-C₄, alcoxy en C₁-C₄, monohydroxyalkyle en C₁-C₄, polyhydroxyalkyle en C₂-C₄, monohydroxyalcoxy en C₁-C₄ ou polyhydroxyalcoxy en C₂-C₄.

Parmi les méta-aminophénols de formule (I) ci-dessus, on peut plus particulièrement citer le méta-aminophénol, le 5-amino 2-méthoxy phénol, le 5-amino 2-(β-hydroxyéthyloxy) phénol, le 5-amino 2-méthyl phénol, le 5-N-(β-hydroxyéthyl)amino 2-méthyl phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, et leurs sels d'addition avec un acide.

D'autres coupleurs utiles sont par exemples, les coupleurs parmi les naphtalènes substitués de formule (II) suivante, et leurs sels d'addition avec un acide : 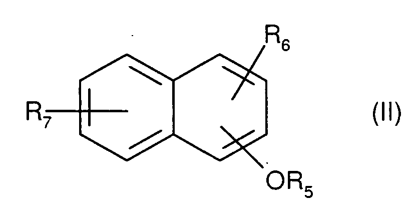 dans laquelle :
- R₅ représente un atome d'hydrogène ou un groupement -CO-R dans lequel R représente un radical alkyle en C₁-C₄ ;
- R₆ représente un atome d'hydrogène, un radical hydroxyle, alkyle en C₁-C₄, ou un groupement -SO₃H ;
- R₇ représente un atome d'hydrogène, ou un radical hydroxyle ; étant entendu qu'au moins un des radicaux R₅ à R₇ étant différent d'un atome d'hydrogène.

Parmi les naphtalènes substitués de formule (II), utilisables à titre de coupleur dans les compositions tinctoriales conformes à l'invention, on peut notamment citer :
- le 1,7-dihydroxy naphtalène,
- le 2,7-dihydroxy naphtalène,
- le 2,5-dihydroxy naphtalène,
- le 2,3-dihydroxy naphtalène,
- le 1-acétoxy 2-méthyl naphtalène,
- le 1-hydroxy 2-méthyl naphtalène,
- l'acide 1-hydroxy 4-naphtalène sulfonique,
et leurs sels d'addition avec un acide.

La ou les méta-phénylènediamines pouvant être utilisées à titre coupleur dans la composition tinctoriale prête à l'emploi conforme à l'invention sont de préférence choisies parmi les composés de formule (III) suivante, et leurs sels d'addition avec un acide : 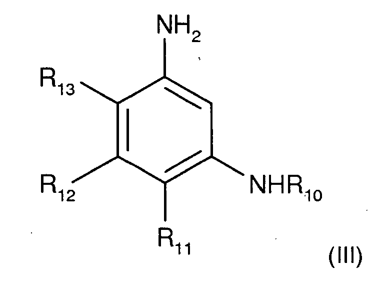 dans laquelle :
- R₁₀ représente un atome d'hydrogène, un radical alkyle en C₁-C₄, monohydroxyalkyle en C₁-C₄ ou polyhydroxyalkyle en C₂-C₄ ;
- R₁₁ et R₁₂, identiques ou différents, représentent un atome d'hydrogène, un radical alkyle en C₁-C₄, monohydroxyalcoxy en C₁-C₄ ou polyhydroxyalcoxy en C₂-C₄ ;
- R₁₃ représente un atome d'hydrogène, un radical alkoxy en C₁-C₄, aminoalkoxy en C₁-C₄, monohydroxyalkoxy en C₁-C₄, polyhydroxyalkoxy en C₂-C₄ ou un radical 2,4-diaminophénoxyalkoxy.

Parmi les méta-phénylènediamines de formule (III) ci-dessus, on peut plus particulièrement citer le 2,4-diamino benzène, le 3,5-diamino 1-éthyl 2-méthoxybenzène, le 3,5-diamino 2-méthoxy 1-méthyl benzène, le 2,4-diamino 1-éthoxybenzène, le 1,3-bis-(2,4-diaminophénoxy) propane, le bis-(2,4-diaminophénoxy) méthane, le 1-(β-aminoéthyloxy) 2,4-diamino benzène, le 2-amino 1-(β-hydroxyéthyloxy) 4-méthylamino benzène, le 2,4-diamino 1-éthoxy 5-méthyl benzène, le 2,4-diamino 5-(β-hydroxyéthyloxy) 1-méthylbenzène, le 2,4-diamino 1-(β,γ-dihydroxypropyloxy) benzène, le 2,4-diamino 1-(β-hydroxyéthyloxy) benzène, le 2-amino 4-N-(β-hydroxyéthyl) amino 1-méthoxy benzène, et leurs sels d'addition avec un acide.

Le ou les méta-diphénols pouvant être utilisés à titre coupleur dans la composition tinctoriale prête à l'emploi conforme à l'invention sont de préférence choisis parmi les composés de formule (IV) suivante, et leurs sels d'addition avec un acide : 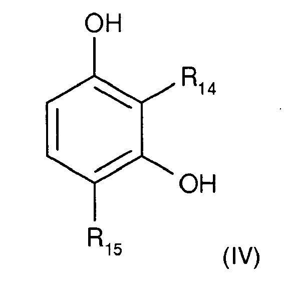 dans laquelle :
- R₁₄ et R₁₅, identiques ou différents, représente un atome d'hydrogène, un radical alkyle en C₁-C₄ ou un atome d'halogène choisi parmi le chlore, le brome ou le fluor.

Parmi les méta-diphénols de formule (IV) ci-dessus, on peut plus particulièrement citer le 1,3-dihydroxy benzène, le 2-méthyl 1,3-dihydroxy benzène, le 4-chloro 1,3-dihydroxy benzène, le 2-chloro 1,3-dihydroxybenzène, et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Au sens de la présente invention, on entend par bio hétéropolysaccharides, des substances synthétisées par fermentation de sucres par des micro-organismes. Les biohétéropolysaccharides ont en particulier souvent des unités choisies parmi les unités mannoses, glucoses et acide glucuroniques ou galacturoniques dans leur chaîne, ces unités étant éventuellement acylées.

On peut notamment citer les gommes de xanthane produites par la bactérie *xanthomonas campestri* et les mutants et variants de celle-ci.

Ces gommes de xanthane ont généralement un poids moléculaire compris entre 1 000 000 et 50 000 000.

On peut aussi citer les gommes de scléroglucane produites par *Sclerotium rolfsii,* les gommes de gellane produites par *Pseudomonas elodea* ou *Sphingomonias,* les gommes de pullulane produites par *Aureobacidium pullulens,* les gommes de Curdlar produites par les alcaligènes de type *Faecalis myxogènes,* les gommes de xanthanes produits par de nombreux organismes dont *Leuconostoc mesenteroides* et *Leuconostoc dextrantum ,* les gommes de grifolane produites par *Grifola frondara,* les gommes de lentinane produites par Lentinus e dodes, les gommes de Schizophyllane produites par *Schizophyllum commine,* les gommes de spirulinane produites par Spirulina sybsyla et les gommes de krestine produites par *Coriates versicolor.*

De préférence, on utilise les gommes de xanthane et de scléroglucane. Encore plus préférentiellement, on utilise les gommes de scléroglucane.

Le biohétéropolysaccharide est en général compris dans la composition de coloration dans des quantités variant de 0.01 à 10 % en poids du poids total de la composition, de préférence entre 0.1 et 4 %.

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

Selon l'invention, les tensioactifs cationiques sont non polymériques, c'est à dire ne sont pas obtenus par polymérisation de monomères autres que les oxyde d'alkylènes ou par greffage de groupes cationiques sur des pigments naturels existants.

A titre d'amines grasses, on peut notamment citer les alkyl amido amines telles que par exemple les alkyl (C₈-C₃₀) amido dialkyl (C₁-C₆)amines et en particulier la stéaramido propyl diméthylamine (MACKINE 301 proposé par MAC INTYRE).

A titre de sels d'ammonium quaternaire, on peut notamment citer, par exemple :
- ceux qui présentent la formule générale (V) suivante : 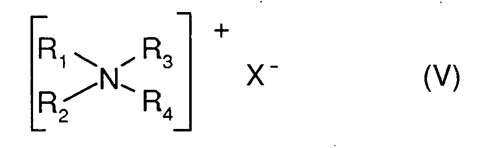 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C₁-C₃₀, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X⁻ est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (VI) suivante : 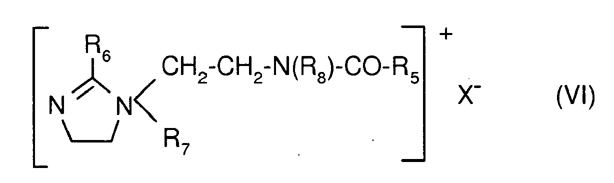 dans laquelle R₅ représente un radical alcényle ou alkyle comportant de 8 à 30 atomes de carbone par exemple dérivés des acides gras du suif ou du coprah, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 2002), le Quaternium-87 (CTFA 2002) ou le Quaternium-83 (CTFA 2002) commercialisés sous les dénominations "VARISOFT^{®}" W575PG par la société GOLDSCHMIDT,
- les sels de diammonium quaternaire de formule (VII) : 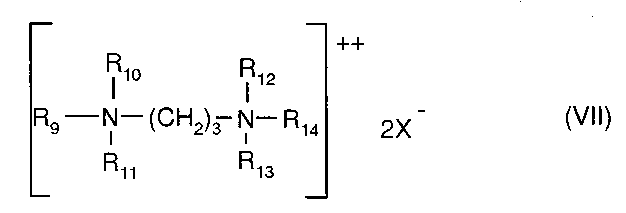 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X⁻ est un anion choisi dans le groupe des halogénures, acétates, phosphates, nitrates, éthylsulfates et méthylsulfates. De tels sels de diammonium quaternaire comprennent notamment le dichlorure de propanesuif diammonium ;
- les sels d'ammonium quaternaire comprenant au moins une fonction ester, tels que ceux de formule (VIII) suivante : 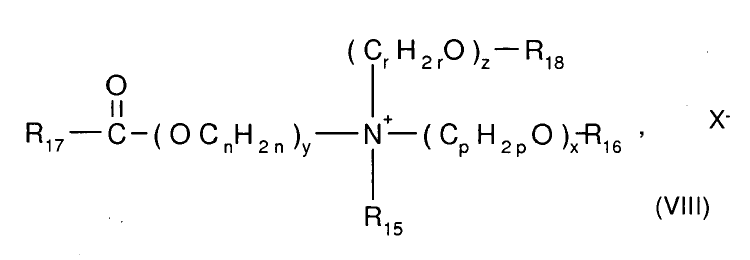 dans laquelle :
   R₁₅ est choisi parmi les radicaux alkyles en C₁-C₆ et les radicaux hydroxyalkyles ou dihydroxyalkyles en C₁-C₆ ;
   R₁₆ est choisi parmi :
      - le radical 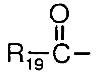
      - les radicaux R₂₀ hydrocarbonés en C₁-C₂₂, linéaires ou ramifiés, saturés ou insaturés,
      - l'atome d'hydrogène,
   R₁₇ est choisi parmi :
      - le radical 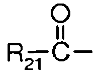
      - les radicaux R₂₂ hydrocarbonés en C₁-C₆, linéaires ou ramifiés, saturés ou insaturés,
      - l'atome d'hydrogène,
   R₁₇, R₁₉ et R₂₁, identiques ou différents, sont choisis parmi les radicaux hydrocarbonés en C₇-C₂₁, linéaires ou ramifiés, saturés ou insaturés ;
   r, n et p, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
   sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés, et plus particulièrement linéaires.

De préférence, R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, n et p, identiques ou différents, valent 2 ou 3, et encore plus particulièrement sont égaux à 2.

L'anion X⁻ est de préférence un halogénure (chlorure, bromure ou iodure) ou un alkyl(C₁-C₄)sulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, n et pt sont égaux à 2 ;
- R₁₆ est choisi parmi :
- le radical 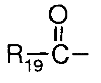
- les radicaux méthyle, éthyle ou hydrocarbonés en C₁₄-C₂₂,
- l'atome d'hydrogène ;
- R₁₈ est choisi parmi :
- le radical 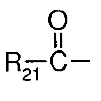
- l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société COGNIS, STEPANQUAT^{®} par la société STEPAN, NOXAMIUM^{®} par la société CECA, REWOQUAT^{®} WE 18 par la société REWO-GOLDSCHMIDT.

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange comprenant 15 à 30 % en poids de méthylsulfate d'acyloxyéthyldihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium comprenant au moins une fonction ester décrits dans les brevets US-A-4874554 et US-A-4137180.

Parmi les sels d'ammonium quaternaire mentionnés ci-dessus, on préfère utiliser ceux répondant à la formule (V) ou de formule (VIII). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium correspondant au QUATERNIUM-70 (CTFA 2002) commercialisé sous la dénomination CERAPHYL^{®} 70 par la société ISP.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltriméthylammonium, le quaternium-83, le quaternium-87, le chlorure de béhénylamidopropyl 2,3-dihydroxypropyl diméthyl ammonium, le chlorure de palmitylamidopropyltriméthylammonium et la stéaramidopropyldiméthylamine.

La composition selon l'invention comprend de préférence le ou les tensioactifs cationiques en une quantité allant de 0,01 à 10 % en poids, de préférence de 0,1 à 4 % en poids par rapport au poids total de la composition.

De préférence, le rapport pondéral entre le ou les tensioactifs cationiques/le ou les biopolysaccharides est supérieur à 1 et idéalement compris entre 1 et 10.

Les tensio-actifs amphotère contenus dans la composition de l'invention sont des tensio-actifs connus en soi dans le domaine de la coloration directe des fibres kératiniques.

Les tensio-actifs amphotères utiles dans la composition de l'invention sont connus de la technique. Ces agents tensioactifs amphotères peuvent être notamment des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL^{®}, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂, désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL^{®} C2M concentré par la société RHODIA.

Les tensioactifs amphotères préférés sont les alkylbétaïnes, les alkylamido alkylbétaïnes et le coamphodiacétate.

Plus préférentiellement, le tensioactif amphotère est une alkylamidoalkylbétaïne.

La composition selon l'invention comprend de préférence le ou les tensioactifs amphotères en une quantité allant de 0,01 à 20 % en poids, de préférence de 5 à 15 % en poids par rapport au poids total de la composition.

La composition de l'invention peut aussi comprendre des tensio-actifs non ioniques par exemple ceux décrits dans "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine, de préférence, les alkylpolyglycosides ou les alcools gras oxyalkylénés.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Pour la teinture des fibres kératiniques humaines, le milieu de teinture est un milieu cosmétique approprié.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, ou zwittérioniques ou leurs mélanges, des polymères anioniques, cationiques, non-ioniques, amphotères, zwitterioniques ou leurs mélanges, des agents épaississants autres que les biohétérosaccharides utiles dans la présente invention, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 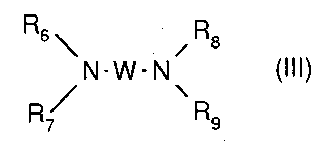 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus et un deuxième compartiment renferme un agent oxydant. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

### EXEMPLES

Les compositions suivantes ont été préparées, les quantités indiquées sauf indication contraire dans des quantités pondérales.

| | Exemple 1 | Exemple 2 |
|---|---|---|
| TOLUENE-2,5-DIAMINE | 0,3 | 0,64 |
| 2-METHYL-5-HYDROXYETHYLAMINOPHENOL | 0,1 | |
| 2,4-DIAMINOPHENOXYETHANOL HCl | | 0,01 |
| 4-AMINO-2-HYDROXYTOLUENE | 0,1 | |
| 6-HYDROXYINDOLE | 0,027 | |
| 2-AMINO-3-HYDROXYPYRIDINE | 0,2 | |
| RESORCINOL | 0,05 | 0,66 |
| m-AMINOPHENOL | 0,01 | 0,085 |
| p-AMINOPHENOL | 0,27 | |
| PENTASODIUM PENTETATE | 2 | 2 |
| AMMONIUM THIOLACTATE | 0,8 | |
| AMMONIUM HYDROXIDE | 11,1 | 11,1 |
| SODIUM METABISULFITE | | 0,71 |
| SCLEROTIUM GUM | 1 | 1 |
| AMODIMETHICONE (and) TRI DECETH-6 (and) CETRIMONIUM CHLORIDE | 1,8 | 1,8 |
| PROPYLENE GLYCOL | 2 | |
| CETEARYL ALCOHOL | 7 | 7 |
| BEHENTRIMONIUM CHLORIDE | 4 | 4 |
| COCAMIDOPROPYLBETAINE | 10,53 | 10,53 |
| AQUA | QS | QS |

Les compositions ci-dessus sont mélangées avec de l'eau oxygénée à 20 volumes (1 volume de composition pour 1,5 volume d'agent oxydant).

Le mélange ainsi obtenu s'applique facilement sur des mèches de cheveux naturels gris à 90% de blancs. Après 30 min de pause, les cheveux sont rincés, lavés avec un shampooing standard et séchés.

La coloration capillaire est évaluée de manière visuelle.

| | Hauteur de ton | Reflet |
|---|---|---|
| Exemple 1 | Blond Foncé | Rouge |
| Exemple 2 | Blond Foncé | Naturel |

Les deux nuances obtenues sont peu sélectives.

Pour chacun de ces exemples, le même résultat est obtenu lorsqu'on remplace les 1% de sclérotium gum par 1% de gomme de xanthane.

## Revendications

1. Composition de coloration des fibres kératiniques comprenant au moins un précurseur de colorant, au moins un biohétéropolysaccharide, au moins un tensio-actif cationique et au moins un tensio-actif amphotère.

2. Composition selon la revendication 1 dans laquelle le précurseur de colorant est choisi parmi les bases d'oxydation et les coupleurs.

3. Composition selon la revendication 1 ou 2 dans laquelle la base d'oxydation est choisie parmi les para-phénylènediamines, les bis-phénylalkylènediamines, les para-aminophénols, les bis-para-aminophénols, les ortho-aminophénols, les bases hétérocycliques et leurs sels d'addition.

4. Composition selon l'une quelconque des revendications précédentes comprenant un coupleur choisi parmi les méta-phénylènediamines, les méta-aminophénols, les méta-diphénols, les coupleurs naphtaléniques, les coupleurs hétérocycliques et leur sels d'addition.

5. Composition selon l'une quelconque des revendications précédentes comprenant au moins une base d'oxydation et un coupleur.

6. Composition selon l'une quelconque des revendications précédentes dans laquelle la quantité de bases d'oxydation est comprise entre 0,001 à 10 % en poids du poids total de la composition tinctoriale et la quantité de coupleur, lorsqu'il est présent, est comprise entre 0,001 et 10 % en poids du poids total de la composition tinctoriale.

7. Composition selon l'une quelconque des revendications précédentes dans laquelle le biohétéropolysaccharide est choisi parmi les gommes de xanthane, les gommes de scléroglucane, les gommes de gellane, les gommes de pullulane, les gommes de Curdlar, les gommes de grifolane, les gommes de lentinane, les gommes de Schizophyllane, les gommes de spirulinane et les gommes de krestine.

8. Composition selon l'une quelconque des revendications précédentes dans laquelle le biohétéropolysaccharide est une gomme de xanthane.

9. Composition selon l'une quelconque des revendications 1 à 7 dans laquelle le biohétéropolysaccharide est une gomme de cléroglucane (sclerotium gum).

10. Composition selon l'une quelconque des revendications précédentes dans laquelle la quantité en biohétéropolysaccharide est comprise entre 0.01 et 10 % en poids du poids total de la composition, de préférence entre 0.1 et 4 %.

11. Composition selon l'une quelconque des revendications précédentes dans laquelle le ou les tensio-actifs cationiques sont choisis parmi les sels d'amines grasses primaires, secondaires ou tertiaires, les sels d'ammonium quaternaire, et leurs mélanges.

12. Composition selon la revendication 11 dans laquelle le tensio-actif cationique est choisi parmi le chlorure de béhényltriméthylammonium, le chlorure de cétyltriméthylammonium, le quaternium-83, le quaternium-87, le chlorure de béhénylamidopropyl 2,3-dihydroxypropyl diméthyl ammonium, le chlorure de palmitylamidopropyltriméthylammonium et la stéaramidopropyldiméthylamine.

13. Composition selon l'une quelconque des revendications précédentes dans laquelle la quantité en tensio-actif cationique est comprise entre 0,01 à 10 % en poids, de préférence de 0,1 à 4 % en poids par rapport au poids total de la composition.

14. composition selon l'une quelconque des revendications précédentes dans laquelle le tensio-actif amphotère est choisi parmi les alkylbétaïnes, les alkylamido alkylbétaïnes et le cocoamphodiacétate.

15. composition selon l'une quelconque des revendications précédentes dans laquelle le ou les tensioactifs amphotères sont présents en quantité variant de 0,01 à 20 % en poids, de préférence de 5 à 15 % en poids par rapport au poids total de la composition.

16. Composition selon l'une quelconque des revendications précédentes dans laquelle le rapport pondéral tensioactifs cationiques/biohétéropolysaccharides est supérieur à 1.

17. Composition selon l'une quelconque des revendications précédentes comprenant au moins un colorant direct.

18. Procédé de coloration des fibres kératiniques dans lequel on applique sur les fibres la composition telle que définie à l'une quelconque des revendications 1 à 17, en présence d'un agent oxydant pendant un temps suffisant pour développer la coloration désirée.

19. Dispositif à plusieurs compartiments dans lequel un premier compartiment renferme la composition tinctoriale telle que définie à l'une quelconque des revendications 1 à 17 et un deuxième compartiment renferme un agent oxydant.

## Claims

1. Composition for dyeing keratin fibres, comprising at least one dye precursor, at least one bioheteropolysaccharide, at least one cationic surfactant and at least one amphoteric surfactant.

2. Composition according to Claim 1, in which the dye precursor is chosen from oxidation bases and couplers.

3. Composition according to Claim 1 or 2, in which the oxidation base is chosen from para-phenylenediamines, bis(phenyl)alkylenediamines, para-aminophenols, bis-para-aminophenols, ortho-aminophenols and heterocyclic bases, and the addition salts thereof.

4. Composition according to any one of the preceding claims, comprising a coupler chosen from meta-phenylenediamines, meta-aminophenols, meta-diphenols, naphthalene-based couplers and heterocyclic couplers, and the addition salts thereof.

5. Composition according to any one of the preceding claims, comprising at least one oxidation base and a coupler.

6. Composition according to any one of the preceding claims, in which the amount of oxidation bases is between 0.001% and 10% by weight relative to the total weight of the dye composition and the amount of coupler, when iL is present, is between 0.001% and 10% by weight relative to the total weight of the dye composition.

7. Composition according to any one of the preceding claims, in which the bioheteropolysaccharide is chosen from xanthan gums, scleroglucan gums, gellan gums, pullulan gums, Curdlar gums, grifolan gums, lentinan gums, schizophyllan gums, spirulinan gums and krestin gums.

8. Composition according to any one of the preceding claims, in which the bioheteropolysaccharide is a xanthan gum.

9. Composition according to any one of Claims 1 to 7, in which the bioheteropolysaccharide is a scleroglucan gum (sclerotium gum).

10. Composition according to any one of the preceding claims, in which the amount of bioheteropolysaccharide is between 0.01% and 10% and preferably between 0.1% and 4% by weight relative to the total weight of the composition.

11. Composition according to any one of the preceding claims, in which the cationic surfactant(s) is (are) chosen from primary, secondary or tertiary fatty amine salts and quaternary ammonium salts, and mixtures thereof.

12. Composition according to Claim 11, in which the cationic surfactant is chosen from behenyltrimethylammonium chloride, cetyltrimethylammonium chloride, quaternium-83, quaternium-87, behenylamidopropyl-2,3-dihydroxypropyldimethylammonium chloride, palmitylamidopropyltrimethylammonium chloride and stearamido-propyldimethylamine.

13. Composition according to any one of the preceding claims, in which the amount of cationic surfactant is between 0.01% and 10% by weigh and preferably from 0.1% to 4% by weight relative to the total weight of the composition.

14. Composition according to any one of the preceding claims, in which the amphoteric surfactant is chosen from alkylbetaines, alkylamidoalkylbetaines and cocoamphodiacetate.

15. Composition according to any one of the preceding claims, in which the amphoteric surfactant(s) is (are) present in an amount ranging from 0.01% to 20% by weight and preferably from 5% to 15% by weight relative to the total weight of the composition.

16. Composition according to any one of the preceding claims, in which the cationic surfactant/biohetero-polysaccharides weight ratio is greater than 1.

17. Composition according to any one of the preceding claims, comprising at least one direct dye.

18. Process for dyeing keratin fibres, in which the composition as defined in any one of Claims 1 to 17 is applied to the fibres, in the presence of an oxidizing agent, for a time that is sufficient to develop the desired colouring.

19. Multi-compartment device, in which a first compartment contains the dye composition as defined in any one of Claims 1 to 17 and a second compartment contains an oxidizing agent.

## Patentansprüche

1. Zusammensetzung zum Färben von Keratinfasern, die mindestens ein Farbstoffvorprodukt eines Oxidationsfarbstoffes, mindestens ein Bioheteropolysaccharid, mindestens einen kationischen grenzflächenaktiven Stoff und mindestens einen amphoteren grenzflächenaktiven Stoff enthält.

2. Zusammensetzung nach Anspruch 1, wobei das Farbstoffvorprodukt eines Oxidationsfarbstoffes unter den Oxidationsbasen und den Kuppler ausgewählt ist.

3. Zusammensetzung nach Anspruch 1 oder 2, wobei die Oxidationsbase unter den para-Phenylendiaminen, Bisphenylalkylendiaminen, para-Aminophenolen, Bis-para-aminophenolen, ortho-Aminophenolen, heterocyclischen Basen und ihren Additionssalzen ausgewählt ist.

4. Zusammensetzung nach einem der vorhergehenden Ansprüche, die einen Kupper enthält, der unter den meta-Phenylendiaminen, meta-Aminophenolen, meta-Dihydroxybenzolen, Naphthalinkupplern und heterocyclischen Kupplern und ihren Additionssalzen ausgewählt ist.

5. Zusammensetzung nach einem der vorhergehenden Ansprüche, die mindestens eine Oxidationsbase und einen Kuppler enthält.

6. Zusammensetzung nach einem der vorhergehenden Ansprüche, wobei der Mengenanteil der Oxidationsbasen im Bereich von 0,001 bis 10 Gew.-% des Gesamtgewichts der Farbmittelzusammensetzung und der Mengenanteil des Kupplers, falls ein Kuppler enthalten ist, im Bereich von 0,001 bis 10 Gew.-% des Gesamtgewichts der Farbmittelzusammensetzung liegt.

7. Zusammensetzung nach einem der vorhergehenden Ansprüche, wobei das Bioheteropolysaccharid unter den Xanthanen, Scleroglucanen, Gellanen, Pullulanen, Curdlanen, Grifolanen, Lentinanen, Schizophyllanen, Spirulinanen und Krestinen ausgewählt ist.

8. Zusammensetzung nach einem der vorhergehenden Ansprüche, wobei das Bioheteropolysaccharid ein Xanthangummi ist.

9. Zusammensetzung nach einem der Ansprüche 1 bis 7, wobei das Bioheteropolysaccharid ein Scleroglucan (Sclerotium gum) ist.

10. Zusammensetzung nach einem der vorhergehenden Ansprüche, wobei der Mengenanteil des Bioheteropolysaccharids im Bereich von 0,01 bis 10 Gew.-% des Gesamtgewichts der Zusammensetzung und vorzugsweise im Bereich von 0,1 bis 4 Gew.-% liegt.

11. Zusammensetzung nach einem der vorhergehenden Ansprüche, wobei der oder die kationischen grenzflächenaktiven Stoffe unter den primären, sekundären oder tertiären Salzen von Fettaminen, quartären Ammoniumsalzen und deren Gemischen ausgewählt sind.

12. Zusammensetzung nach Anspruch 11, wobei der kationische grenzflächenaktive Stoff unter Behenyltrimethylammoniumchlorid, Cetyltrimethylammoniumchlorid, Quaternium-83, Quaternium-87, Behenylamidopropyl-2,3-dihydroxypropyl-dimethyl-ammoniumchlorid, Palmitylamidopropyltrimethylammoniumchlorid und Stearamidopropyldimethylamin ausgewählt ist.

13. Zusammensetzung nach einem der vorhergehenden Ansprüche, wobei der Mengenanteil des kationischen grenzflächenaktiven Stoffes im Bereich von 0,01 bis 10 Gew.-% und vorzugsweise 0,1 bis 4 Gew.-%, bezogen auf das Gesamtgewicht der Zusammensetzung, liegt.

14. Zusammensetzung nach einem der vorhergehenden Ansprüche, wobei der amphotere grenzflächenaktive Stoff unter den Alkylbetainen, Alkylamidoalkylbetainen und Cocoamphodiacetat ausgewählt ist.

15. Zusammensetzung nach einem der vorhergehenden Ansprüche, wobei der oder die amphoteren grenzflächenaktiven Stoffe in einem Mengenanteil von 0,01 bis 20 Gew.-% und vorzugsweise 5 bis 15 Gew.-%, bezogen auf das Gesamtgewicht der Zusammensetzung, enthalten sind.

16. Zusammensetzung nach einem der vorhergehenden Ansprüche, wobei das Gewichtsverhältnis kationische grenzflächenaktive Stoffe/Bioheteropolysaccharide über 1 liegt.

17. Zusammensetzung nach einem der vorhergehenden Ansprüche, die mindestens einen Direktfarbstoff enthält.

18. Verfahren zum Färben von Keratinfasern, bei dem die Zusammensetzung, wie sie in einem der Ansprüche 1 bis 17 definiert ist, während einer Zeitspanne, die für die Bildung der gewünschten Farbe ausreichend ist, in Gegenwart eines Oxidationsmittels auf die Fasern aufgebracht wird.

19. Vorrichtung mit mehreren Abteilungen, wobei eine erste Abteilung die Farbmittelzusammensetzung, wie sie in einem der Ansprüche 1 bis 17 definiert ist, und eine zweite Abteilung ein Oxidationsmittel enthält.
